# EUROPÄISCHE PATENTSCHRIFT

(11) **EP 2 588 046 B1**
(45) Veröffentlichungstag und Bekanntmachung des Hinweises auf die Patenterteilung: **08.07.2015**
(21) Anmeldenummer: 11758085.2
(22) Anmeldetag: 25.05.2011
(51) Int. Cl.: A61F 5/37

(54) **Orthese mit wenigstens einer textilen Bandage**
Orthosis with at least one textile bandage
Orthèse comprenant au moins un bandage en textile

(30) Priorität: 01.07.2010 DE 102010026240
(43) Veröffentlichungstag der Anmeldung: 08.05.2013
(73) Patentinhaber: Otto Bock HealthCare GmbH, 37115 Duderstadt (DE)
(72) Erfinder: REINHARDT, Holger, 47906 Kempen (DE); HORMES, Helle, 37154 Northeim (DE)
(74) Vertreter: Lins, Edgar
(86) Internationale Anmeldenummer: PCT/DE2011/001106
(87) Internationale Veröffentlichungsnummer: WO 2012/006984

(56) Entgegenhaltungen:
- WO-A1-2009/017442
- US-A- 3 008 466
- US-A- 3 404 680
- US-A- 5 403 268
- US-A- 5 713 837
- US-A1- 2008 039 757

## Beschreibung

Die Erfindung betrifft eine Orthese mit wenigstens einem Halteelement zum Anlegen an ein Körperteil und Befestigen des Halteelements mit wenigstens einem Befestigungsmittel, mit dem zwei Enden des Halteelements am Körperteil anliegend unter Spannung miteinander verbindbar sind, wobei das wenigstens eine Halteelement ohne ein stabiles Formteil ausgebildet ist, und wobei das Halteelement wenigstens ein elastisches Aufspreizteil aufweist, das Halteelement in eine solche Ausgangsform vorspannt, dass die Enden des Halteelements vom Körperteil abstehen und dass mit dem Befestigungsmittel die Enden des Halteelements gegen die Vorspannung des Aufspreizteils an das Körperteil anlegbar sind.

Orthesen werden in zahlreichen Ausführungsformen hergestellt und verwendet. Insbesondere haben sich Orthesen mit textilen Bandagen alshalteelement bei Schlaganfallspatienten bewährt, die nach dem Schlaganfall halbseitig gelähmt sind. Für diese Patienten ist es wichtig, dass auf der gelähmten Körperhälfte Schulter, Arm und Hand fixiert sind, um nicht durch unkontrollierte Bewegungen der Gefahr von Verletzungen ausgesetzt zu sein.

Ein bekanntes Orthesensystem für Hemiparese-Patienten besteht aus einem Schulterteil, einem Stützteil für das Ellbogengelenk und ggf. einer Handgelenksorthese. Das Schulterteil wird dabei mit Befestigungsbändern um den Oberkörper geschlungen und dabei unter der Achselhöhle auf der gesunden Seite des Körpers hindurchgeführt. Das Schulterteil übergreift einen Teil des Oberarms und ist mit einem Befestigungsstreifen mit dem Ellbogenteil verbunden. Die bekannte Orthese ist wirksam und preisgünstig, da sie aus preiswerten Textilbandagen besteht. Dabei ist die Textilbandage, die die Schulter übergreift, durch entsprechende Abnäher so ausgebildet, dass sie sich an die Schulterform und das Übergreifen des Oberarms in ihrer Form anpassen kann. Die Verwendung von Textilbandagen hat allerdings den Nachteil, dass diese ein schlaffes Ausgangsmaterial darstellen, das an die Körperteile angelegt werden muss, um dann mit den flexiblen Befestigungsmitteln, die regelmäßig aus mit Klettverschlüssen versehenen Textilbändern bestehen, befestigt zu werden. Das Anlegen der Orthese erfordert daher eine Hilfsperson, sodass der Patient erst mobil wird, wenn eine Hilfsperson zum Anlegen der Orthese zur Verfügung gestanden hat.

Eine Orthese der eingangs erwähnten Art in Form einer Schulter-Oberarm-Orthese ist durch US 5 403 268 bekannt. Das Schulterteil ist dabei aus einem im Ausgangszustand flach liegenden Streifen gebildet, der durch einen unter der Achse der gesunden Schulterseite hindurch geführten Befestigungsriemen so geformt wird, dass er sich an die Schulterkontur anlegt und so einen Schultersattel bildet, der als Basis für die Armunterstützung dient. Der Sattel besteht aus einem steifen, aber flexiblen Material, sodass er im angelegten Zustand die Funktion eines an das Körperteil angepassten Formteils ausübt und die Stützfunktion bewirkt. Das Anlegen einer derartigen Orthese erfordert die Ausübung einer erheblichen Kraft zur Verformung des Streifenmaterials zu dem Schultersattel und ist daher durch den Patienten selbst nicht möglich.

Ähnliche Orthesen sind durch US 5 713 837 und US 2008/0039757 A1 bekannt.

US 3 008 466 offenbart ein durch eine Schnürung geformtes Stützteil, das beispielsweise als Schiene für einen Arm dient.

US 3 404 680 offenbart eine aus streifenförmigen Riemen gebildete Schulterschlinge, die kein gesondertes Halteelement aufweist.

Es besteht daher ein Bedürfnis, Orthesensysteme einhändig anlegen zu können, um es beispielsweise Hemiparese-Patienten zu ermöglichen, die für die gelähmte Körperseite benötigte Orthese selbst anzulegen und so ein erhebliches Maß an Selbständigkeit zurückzugewinnen.

Selbstverständlich gibt es weitere Fälle, für die das einhändige Anlegen einer Orthese von Vorteil wäre. Die entsprechende Orthese kann dabei auch eine Beinarthese, Fußgelenkorthese o .ä. sein.

Es ist bekannt, einhändig anzulegende Orthesen mit Formteilen auszubilden, die an die Körperteile, an die die Orthese angelegt wird, angepasst sind. Derartige Orthesen sind jedoch aufwändig in ihrer Herstellung, da sie regelmäßig an den speziellen Patienten in Einzelanfertigung angepasst werden müssen. Darüber hinaus sind die Formteile nicht so komfortabel zu benutzen wie textile Bandagen, die unauffällig unter der Kleidung getragen werden können.

Der vorliegenden Erfindung liegt daher die Aufgabe zugrunde, eine Orthese so auszubilden, dass sie einhändig angelegt und befestigt werden kann, preiswert herstellbar ist und unauffällig zu tragen ist.

Zur Lösung dieser Aufgabe ist erfindungsgemäß eine Orthese der eingangs erwähnten Art dadurch gekennzeichnet, dass das Aufspreizteil eine Vorspannkraft aufweist, die lediglich ausreicht, um das Halteelement in die Ausgangsform aufzuspannen, sodass das Aufspreizteil nicht die Funktion eines an das Körperteil angepasste Formteil und keine Stützfunktion für das Körperteil ausübt.

Die erfindungsgemäße Orthese verwendet somit weiterhin Haltelemente, die nicht vorgeformt sind und textile Bandagen sein können, wie sie sich in vielen Anwendungsfällen bewährt haben. Die Stützfunktion der Orthese wird daher von dem jeweiligen schlaffen Halteelement ohne ein an die Umfangsform des Körperteils angepasstes stabiles Formteil ausgeübt. Erfindungsgemäß ist das Halteelement mit wenigstens einem elastischen Aufspreizteil versehen, durch das das Halteelement in eine Ausgangsform vorgespannt wird, in der die Enden des Halteelements von dem Körperteil abstehen. Dadurch ist es möglich, einhändig das Halteelement an dem Körperteil anzulegen und das vorzugsweise flexible Befestigungsmittel zu ergreifen, mit dem das Halteelement an dem Körperteil dadurch festgelegt wird, dass die Enden des Halteelements gegen die Vorspannung des Aufspreizteils an das Körperteil angelegt werden.

Das erfindungsgemäße Aufspreizteil, das vorzugsweise auf der körperfernen Seite einer textilen Bandage aufgebracht ist, bringt die somit an sich schlaffe textile Bandage in eine Ausgangsform, in der die textile Bandage in sich sortiert ist und zum Spannen mit dem Befestigungsmittel auch vorbereitet sein kann, wie dies nachstehend noch näher erläutert wird. Die Ausgangsform der textilen Bandage kann daher insbesondere tunnelförmig sein, sodass die Bandage in der Ausgangsform beispielsweise über die Schulter gelegt werden kann oder in die Bandage das Ellbogengelenk oder der Unterarm einlegbar sind. Die Ausgangsform ergibt sich dabei aus der entsprechenden Vorbereitung der textilen Bandage, nicht durch das elastische Aufspreizteil, das lediglich für die nötige Vorspannung sorgt, nicht jedoch als an den Körperteil angepasstes Formteil ausgebildet ist.

Eine geeignete Formgebung des Aufspreizteils kann sich lediglich daraus ergeben, dass die textile Bandage an geeigneten Punkten, vorzugsweise in der Nähe des Randes der Bandage, durch das Aufspreizteil in die Ausgangsform aufgespannt wird.

Das Aufspreizteil hat dabei nicht die Funktion eines an den Körperteil angepassten Formteils und kann daher mit einer sehr schwachen Vorspannkraft ausgebildet sein. Die Vorspannkraft muss lediglich ausreichen, um die textile Bandage in die Ausgangsform aufzuspannen. Irgendeine Stützfunktion für den Körperteil muss dadurch nicht erfolgen. Demgemäß kann die textile Bandage auch in einfacher Weise und ohne großen Kraftaufwand gegen die Vorspannung des Aufspreizteils an dem Körperteil festgelegt werden.

In einer alternativen Ausführungsform kann insbesondere für sehr einfache Orthesen das Aufspreizteil auch ohne eine textile Bandage verwendet werden, wenn sie zur Anlage an dem betreffenden Körperteil mit wenigstens einem Polsterstück versehen ist. Das Aufspreizteil ist kein an die Umfangsform des Körperteils angepasstes Formteil und hat keine Formstabilität eines Formteils, sondern dient lediglich der Sicherstellung einer im Wesentlichen flachen Ausgangsform, aus der das Aufspreizteil mittels der Befestigungsmittel zum Körperteil hingezogen wird, um an dem Körperteil anzuliegen.

Das Befestigungsmittel wird dabei in Verbindung mit dem Halteelement regelmäßig eine Umschlingung des jeweiligen Körperteils bewirken, um das Halteelement an dem Körperteil festzulegen, sodass die durch die Orthese angestrebte Stütz- bzw. Haltefunktion erzielt wird.

Das Befestigungsmittel ist vorzugsweise ein textiles Befestigungsband, das mit Klettverschlusselementen die Befestigung bewirken kann. Das Befestigungsband kann dabei aus einem elastischen oder vorzugsweise nicht elastischen textilen Material bestehen.

Zum Festlegen des Halteelements ist das Befestigungsband zweckmäßigerweise mit wenigstens zwei Punkten des Halteelements verbindbar. Es ist allerdings auch möglich, ein Befestigungsband nur an einer Stelle mit dem Halteelement zu verbinden und das Befestigungsband zum Umschlingen des Körperteils mit dem Halteelement vorzusehen, wobei das Befestigungsband auf sich selbst festgelegt wird, beispielsweise mittels eines Klettverschlusses.

In einer aufgrund einer leichteren Handhabung bevorzugten Ausführungsform ist das Befestigungsband an einem der Befestigungspunkte mit dem Halteelement durch eine Öse geführt und um 180° umgelenkt. Dabei kann mit Vorteil der umgelenkte Abschnitt mit einer Verbreiterung unverlierbar in der Öse gehalten sein. Auf diese Weise ergibt sich eine vorteilhafte Festlegung des Befestigungsbandes für die nicht angelegte Orthese, die durch das Aufspreizteil in der aufgespreizten Form vorliegt. Das Befestigungsband kann dabei die Ausgangsform des Halteelements, das durch das elastische Aufspreizteil aufgespreizt ist, bestimmen und das Halteelement beispielsweise auf der offenen Seite einer tunnelförmigen Ausgangsform verschließen. Das entsprechende Halteelement kann dann dadurch beispielsweise an einem Arm angelegt werden, dass die Hand und der Unterarm in den Tunnel eingeführt wird bzw. das Halteband mit seinem tunnelförmigen Kanal über die gelähmte Hand und den Unterarm gezogen wird, um beispielsweise an dem Ellbogengelenk positioniert zu werden. Durch Anziehen der Befestigungsbänder kann dann die Fixierung an dem Körperteil, beispielsweise dem Ellbogengelenk, erfolgen, indem das Befestigungsband mittels des Klettverschlusses auf sich selbst oder an einer geeigneten Stelle des Halteelements fixiert wird.

Die Erfindung soll im Folgenden anhand von in der Zeichnung dargestellten Ausführungsbeispielen näher erläutert werden. Es zeigen:
- Figur 1: eine Teil-Frontansicht eines Patienten mit einer angelegten Hemiparese-Orthese;
- Figur 2: eine Ansicht von schräg vorne auf die Darstellung gemäß Figur 1;
- Figur 3: eine Ansicht von hinten auf die Darstellung gemäß Figur 1;
- Figur 4: eine Ansicht einer Ellbogen-Orthese als Einzelteil;
- Figur 5: eine Ansicht einer Handgelenksorthese als Einzelteil;
- Figur 6: eine Ansicht der an den Arm eines Patienten angelegten Handgelenksorthese gemäß Figur 5;
- Figur 7: eine Ansicht einer zur Figur 4 alternativen Ausführungsform eines Halteelements einer Ellbogen-Orthese;
- Figur 8: einen Schnitt durch das Halteelement gemäß Figur 7.

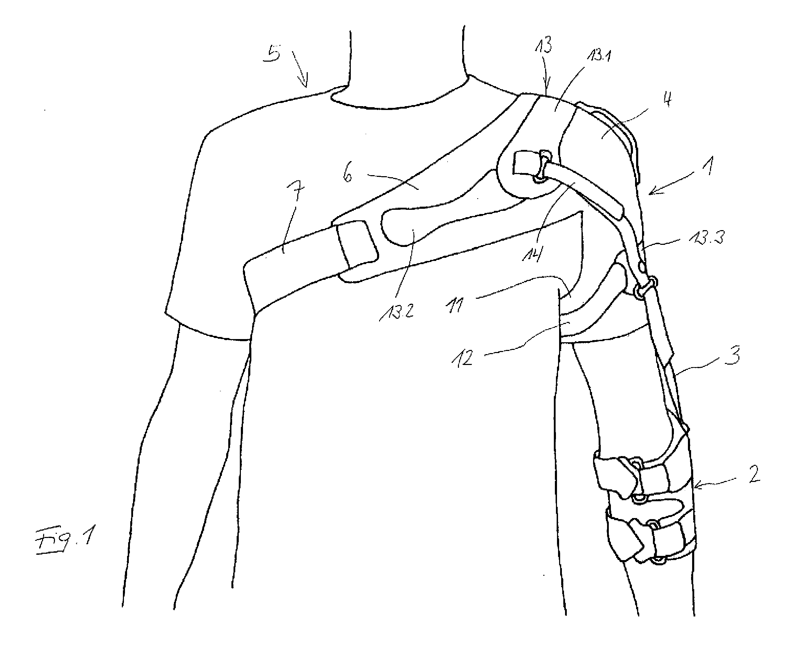

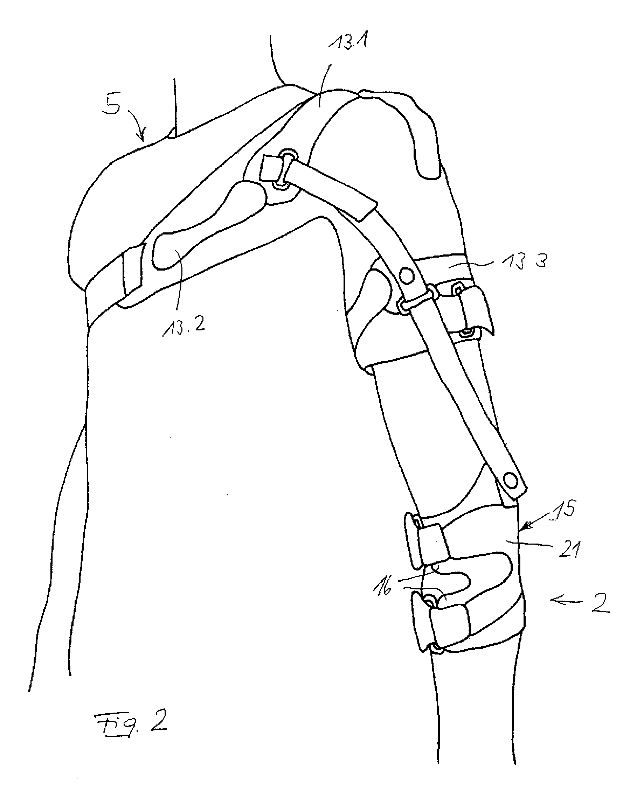

Die in den Figuren 1 bis 3 dargestellte Hemiparese-Orthese besteht aus einem Schultergelenksteil 1 und einem Ellbogenteil 2, die über ein Verbindungsband 3 miteinander verbunden sind.

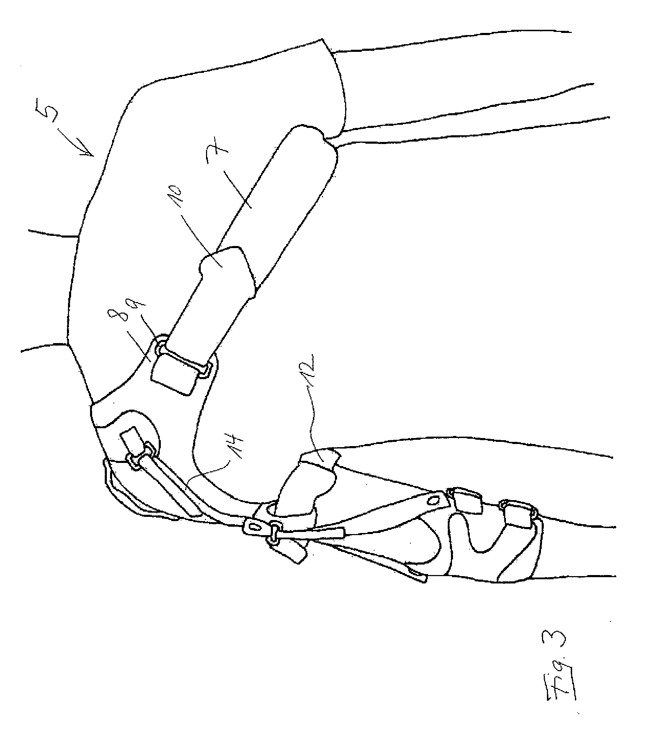

Das Schultergelenksteil 1 besteht aus einer textilen Bandage als Halteelement 4, die von oben über das Schultergelenk, die Schultergelenkskugel und den Oberarm eines Patienten 5 ragt. Ein größerer, auf der Vorderseite des Patienten konisch zulaufender Arm 6 erstreckt sich über den oberen Brustbereich des Patienten 5. An seinem Ende ist ein Befestigungsband 7als flexibles Befestigungsmittel befestigt. Wie die Figuren 1 und 3 verdeutlichen, verläuft das Befestigungsband 7 um den Körper des Patienten 5 herum und ist unterhalb der Achselhöhle auf der gesunden Seite des Patienten geführt. Auf der Rückseite des Patienten (Figur 3) verläuft das Befestigungsband 7 schräg nach oben zu einem Arm 8 der textilen Bandage 4. Dort ist das Befestigungsband durch eine Öse 9 hindurchgeführt und auf sich selbst mit einem dreieckigen, pfeilförmigen verbreiterten Ende 10 festgelegt. Hierzu ist die betreffende Seite des Befestigungsbandes 7 und des pfeilförmigen Endes 10 mit entsprechenden Teilen eines Klettverschlusses versehen.

Die textile Bandage 4 erstreckt sich ferner mit einem weiteren Arm 11 unter die Achselhöhle auf der gelähmten Seite des Patienten 5 und ist dort ebenfalls mit einem Befestigungsband 12 als flexibles Befestigungsmittel versehen. Das Befestigungsband 12 verläuft um den Oberarm auf dessen Innenseite herum und liegt nach der Umschlingung des Oberarms von der Rückseite des Patienten 5 ausgehend auf der textilen Bandage 4 an und ist dort fixiert, beispielsweise wieder mit einem Klettverschluss.

Auf der vom Körper des Patienten 5 abgewandten Seite ist die textile Bandage mit mehreren Aufspreizteilen 13 versehen, die auf der textilen Bandage aufgenäht sein können. Ein erstes Aufspreizteil 13.1 erstreckt sich über die Schulteroberseite von der Vorderseite zur Rückseite des Patienten 5. Auf der Rückseite des Patienten 5 endet das Aufspreizteil 13.1 im Bereich des dortigen Arms 8, wie Figur 3 zeigt. Auf der Vorderseite schließt sich an das Aufspreizteil 13.1 ein weiteres streifenförmiges Aufspreizteil 13.2 an, das sich in den Bereich des Arms 6 der textilen Bandage erstreckt und in der Nähe der Befestigung des Befestigungsbandes 7 an diesem Arm 6 endet.

Ein weiteres Aufspreizteil 13.3 ist im Oberarmbereich vorgesehen und verläuft senkrecht zur Längserstreckung des Oberarmknochens des Patienten 5. Es ist erkennbar, dass das Befestigungsband 12 im Bereich des Aufspreizteils 13.3 befestigt ist. Zwischen den Aufspreizteilen 13.1 und 13.3 verlaufen ferner Einstellbänder 14, mit denen die Krümmung der textilen Bandage 4 an den Patienten 5 in gewisser Weise anpassbar ist.

Die Aufspreizteile 13 bestehen aus flachen, dünnen und elastischen Kunststoffstreifen, die aus einem flachen Kunststoffmaterial ausgeschnitten sind. Soweit sie durch die Form der textilen Bandage 4 verformt werden, haben sie die Tendenz, in die flache Ausgangsposition zurückzukehren. Dadurch stehen die Arme 6, 8 und 11 etwa geradlinig von der übrigen textilen Bandage 4, wenn die Orthese vom Körper des Patienten 5 abgenommen wird und über die Befestigungsbänder 7, 12 kein Zug zwischen den Armen 6, 8 bzw. auf den Arm 11 ausgeübt wird. Der zentrale Abschnitt der textilen Bandage 4, von dem die Arme 6, 8 und 11 ausgehen, ist durch Abnäher etwa an die Form der Schulter und des Oberarms angepasst, wobei dabei nur eine ungefähre Anpassung an die typische Form eines Schultergelenks und Oberarm vorgenommen wird. Die Festlegung der textilen Bandage 4 an den Körper des Patienten 5 erfolgt über die Arme 6, 8, 11 und mit den Befestigungsbändern 11, 12, die einerseits den Oberkörper und andererseits den Oberarm des Patienten 5 umschlingen.

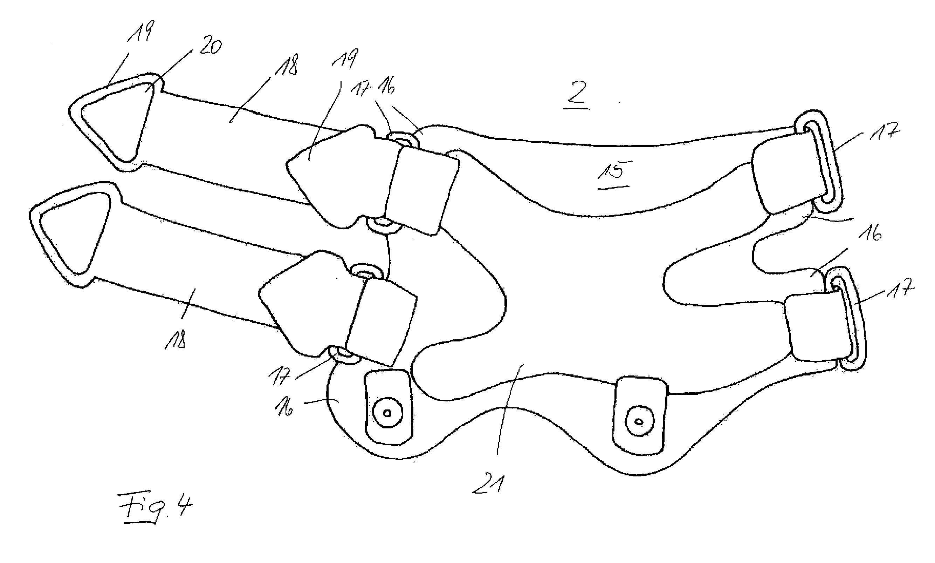

Das Ellbogenteil besteht ebenfalls aus einer flachen textilen Bandage als Halteelement 15, die durch ein im Wesentlichen viereckiges textiles Stück mit vier Armen 16 gebildet ist, wie dies insbesondere Figur 4 verdeutlicht. An den vier Armen 16 ist jeweils eine Öse 17 befestigt. Zwei Befestigungsbänder 18 als flexible Befestigungsmittel sind als unelastische Bänder mit pfeilförmigen verbreiterten Enden 19 an beiden Enden ihrer Länge abgeschlossen. Die pfeilförmigen Enden 19 sind auf ihrer Innenseite mit einem dreieckförmigen Klettverschlussteil 20 versehen, das ein Klettverschlussgegenstück zu der inneren Oberfläche des Befestigungsbands 18 bildet. Beispielsweise kann die innere Oberfläche der Befestigungsbänder mit einem Schlaufenmaterial ausgebildet sein, während die Klettverschlussteile 20 ein zugehöriges Hakenmaterial aufweisen. Die innere Oberfläche der Befestigungsbänder 18 ist diejenige, auf die die pfeilförmigen Enden 19 zum Zwecke ihrer Fixierung zurücklegbar sind. Die innere Oberfläche der Befestigungsbänder 18 befindet sich somit auf derselben Seite der Befestigungsbänder 18 wie die Klettverschlussteile 20, wie dies Figur 4 verdeutlicht.

In der Darstellung der Figur 4 sind die Befestigungsbänder 18 nur mit einem ihrer pfeilförmigen Enden durch eine Öse 17 hindurchgezogen. Dadurch üben die Befestigungsbänder 18 keinen Zug auf die Arme 16 der textilen Bandage 15 aus. Diese wird somit durch ein Aufspreizteil 21 in eine flachliegende Ausgangsform vorgespannt. Wenn die in Figur 4 freien pfeilförmigen Enden 19 der Befestigungsbänder 18 durch die in Figur 4 freien Ösen 17 hindurchgesteckt werden, entsteht ein Zug durch die Länge der Befestigungsbänder 18 auf die textile Bandage 15. Diese wird dadurch in eine U-förmige Form gegen die Vorspannung des Aufspreizteils 21 gebogen. Die jeweiligen Arme 16 werden dann durch ein geradlinig gespanntes Befestigungsband 18 miteinander verbunden. Die Arme 16 bilden Enden der textilen Bandage 15, deren Öffnungswinkel durch die Befestigungsbänder 18 deutlich größer als die Breite des Arms des Patienten 5 im Bereich des Ellbogens ist. Somit bildet die U-förmig gekrümmte textile Bandage 15 mit den Befestigungsbänder 18 einen Einschlupfkanal, in den die Hand und der herunterhängende Arm des Patienten 5 auf der gelähmten Seite bequem eingeführt werden kann, indem die Bandage 15 über die Hand und den Unterarm des Patienten 5 gezogen wird.

Zum Anlegen der Orthese gemäß den Figuren 1 bis 3 wird daher das Ellbogenteil 2 in die beschriebene Anziehstellung gebracht und hängt von dem Schultergelenksteil 1 herunter. Durch Überziehen des Ellbogenteils über Hand und Unterarm des Patienten 5 gelangt das Ellbogenteil in die Position am Ellbogen des Patienten 5. In dieser Position kann das Schultergelenksteil über Schulter und Oberarm des Patienten 5 gelegt werden. Aufgrund der Aufspreizteile 13 kann durch das Befestigungsband 7 mit der textilen Bandage 4 eine Schlaufe gebildet werden und der gesunde Arm durch diese Schlaufe hindurchgesteckt werden, sodass das Befestigungsband 7 in der in den Figuren 1 bis 3 dargestellten Befestigungsstellung unter der Achselhöhle des gesunden Arms des Patienten 5 um den Oberkörper umlaufen kann. Durch ein anschließendes Anziehen des Befestigungsbands 12 zur Umschlingung des Oberarms wird das Schultergelenksteil 8 in der Gebrauchsstellung festgelegt. Anschließend werden die beiden Befestigungsbänder 18 des Ellbogenteils festgezogen und am Ellbogen abschließend positioniert.

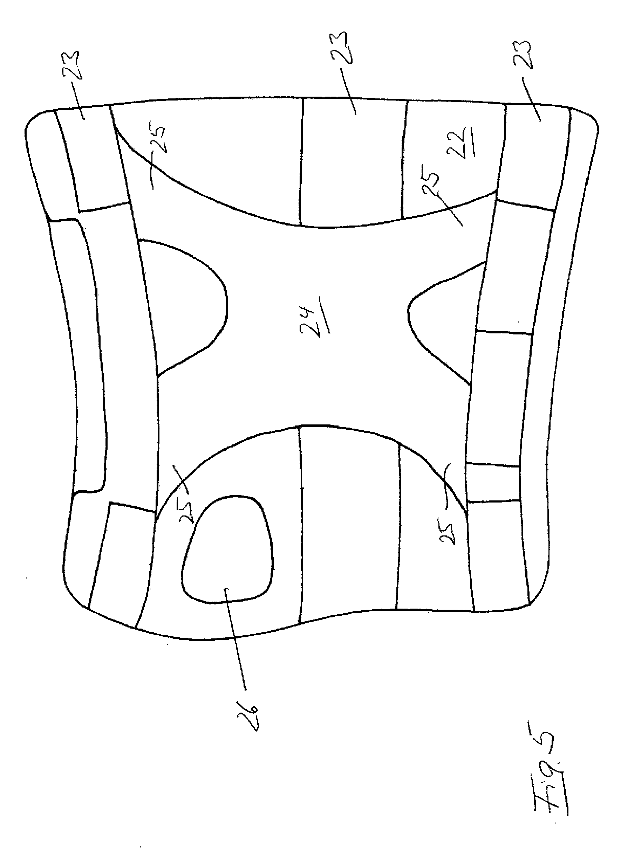

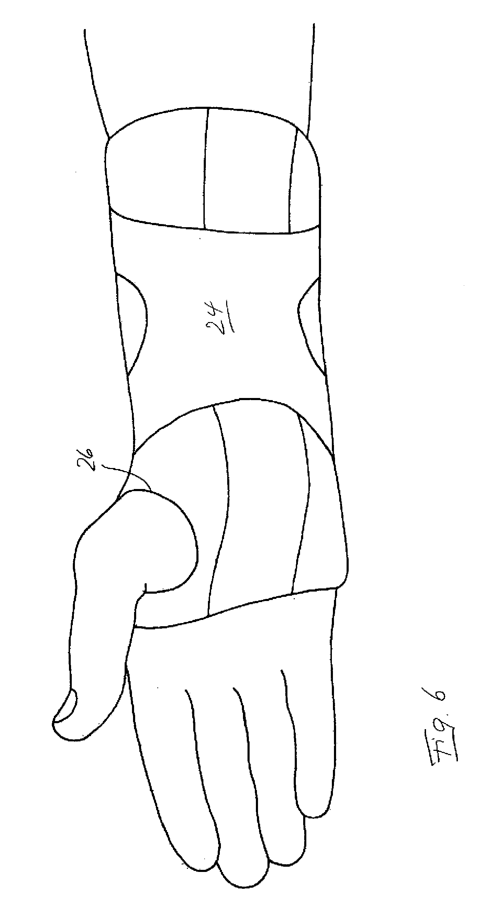

Ergänzend kann dann noch eine Handgelenksorthese angebracht werden, wie sie in Figur 5 im Ausgangszustand und in Figur 6 im angelegten Zustand dargestellt ist.

Die Handgelenksorthese besteht gemäß Figur 5 aus einer im Wesentlichen rechteckigen textilen Bandage als Halteelement 22, die mit drei textilen, aufgenähten Längsstreifen 23 verstärkt ist.

Ein im Wesentlichen X-förmiges Aufspreizteil 24 erstreckt sich mit vier Armen 25 bis zu zwei Verstärkungsstreifen 23 am Längsrand der Bandage 22. Eine Durchgangsöffnung 26 erlaubt das Durchstecken eines Daumens der gelähmten Hand des Patienten 5, wie dies in Figur 6 verdeutlicht ist.

In Figur 6 ist nicht dargestellt der Verschluss der Handgelenksorthese mit Befestigungsstreifen o. dgl. Dieser Verschluss ist in gleicher Weise wie beim Ellbogenteil 2 gemäß Figur 4 möglich.

Figur 6 verdeutlicht, dass die Handgelenksorthese so angelegt wird, dass das Aufspreizteil 24 im Wesentlichen auf der Handgelenksinnenseite zu liegen kommt. Dies ist jedoch nicht notwendigerweise der Fall. Auch ein Anlegen der Handgelenksorthese von außen nach innen ist ohne weiteres möglich und einhändig auszuführen.

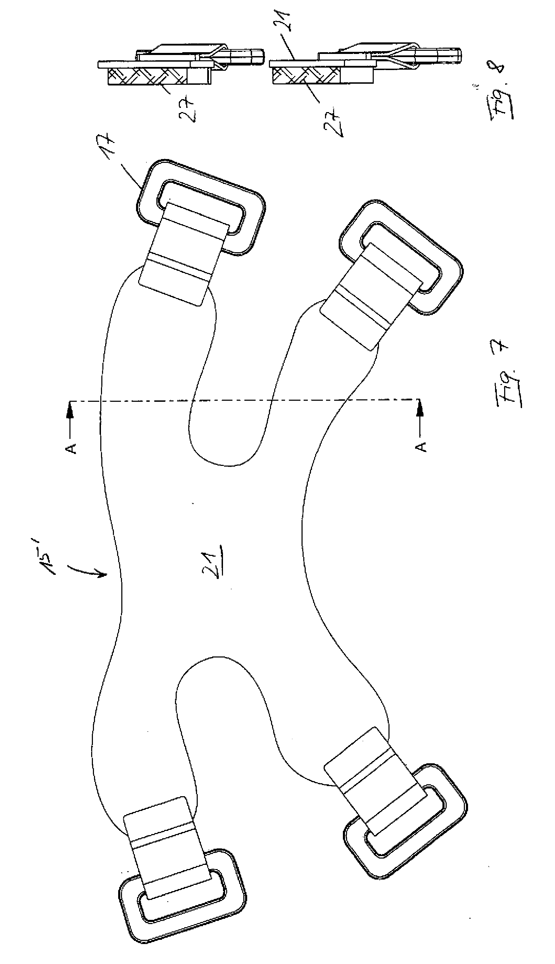

Figur 7 zeigt eine der Ellbogen-Orthese gemäß 4, bei der das Halteelement 15' durch das Aufspreizteil 21 selbst gebildet ist. Die Ösen 17 sind somit unmittelbar an dem Aufspreizteil 21 befestigt.

Figur 8 verdeutlicht, dass das Aufspreizteil 21 auf seiner zum Körperteil hin gerichteten Seite mit Polsterstücken 27 versehen ist, sodass eine bequeme Anlage des Aufspreizteils 21 am Körperteil möglich ist. Die Form des Aufspreizteils 21 entspricht, wie der Vergleich mit Figur 4 zeigt, der Form des Aufspreizteils 21 an der textilen Bandage 15 in Figur 4. Da die textile Bandage 15 in Figur 4 lediglich die Aufgabe hat, die Verbindung zu den Ösen 17 herzustellen, besteht die Modifikation der Figur 7 lediglich darin, die Ösen 17 unmittelbar an den Armen des Aufspreizteils 21 zu befestigten. Das Anlegen der Ellbogen-Orthese gemäß den Figuren 7 und 8 geschieht in genau der gleichen Weise wie bei der Ellbogen-Orthese gemäß Figur 4.

Die dargestellten Ausführungsbeispiele verdeutlichen, dass mit den erfindungsgemäßen Aufspreizteilen 13, 21 und 24 auch für kompliziertere Formen von textilen Bandagen 4, 15 und 22 ein einhändiges Anlegen der Orthesen möglich ist, sodass der Patient 5 selbst seine Orthese anlegen kann und nicht auf die Hilfe einer Pflegeperson angewiesen ist. Im Rahmen der vorliegenden Erfindung kann das Halteelement 4, 5, 22 mit einer formstabilen Verstärkung versehen sein, die jedoch nicht in Umfangsrichtung an die Form des Körperteils angepasst ist. Beispielsweise kann bei einer Handgelenksorthese eine in Längsrichtung des Arms und der Hand verlaufende Schiene in das Aufspreizteil 21 oder in die textile Bandgage 15 eingearbeitet sein.

Selbstverständlich lässt sich das in den Beispielen dargestellte Prinzip der Orthesen auch auf Fußgelenksorthesen, Beinorthesen oder auch Rumpforthesen übertragen. Besonders verteilhaft ist in allen Fällen, wenn die Befestigungsbänder 7, 12, 18 in der dargestellten Weise unverlierbar in Ösen 9, 17 gehalten werden und auch dadurch eine Vorformung der textilen Bandage bewirken.

## Patentansprüche

1. Orthese mit wenigstens einem Halteelement (4, 15, 22), das ohne ein an eine Umfangsform des Körperteils angepasstes stabiles Formteil ausgebildet ist, zum Anlegen an ein Körperteil und Befestigen des Halteelements (4, 15, 22) mit wenigstens einem Befestigungsmittel (7, 12, 18), mit dem zwei Enden des Halteelements (4, 15, 22) am Körperteil anliegend unter Spannung miteinander verbindbar sind, wobei das Halteelement (4, 15, 22) wenigstens ein elastisches Aufspreizteil (13, 21, 24) aufweist, das das Halteelement (4, 15, 22) in eine solche Ausgangsform vorspannt, dass die Enden des Halteelements (4, 15, 22) vom Körperteil abstehen und dass mit dem Befestigungsmittel (7, 12, 18) die Enden des Halteelements (4, 15, 22) gegen die Vorspannung des Aufspreizteils (13, 21, 24) an das Körperteil anlegbar sind, **dadurch gekennzeichnet, dass** das Aufspreizteil eine Vorspannkraft aufweist, die lediglich ausreicht, um das Halteelement in die Ausgangsform aufzuspannen, sodass das Aufspreizteil nicht die Funktion eines an das Körperteil angepassten Formteils und keine Stützfunktion für das Körperteil ausübt.

2. Orthese nach Anspruch 1, **dadurch gekennzeichnet, dass** das Befestigungsmittel (7, 12, 18) ein Befestigungsband ist.

3. Orthese nach Anspruch 2, **dadurch gekennzeichnet, dass** das Befestigungsband (7, 12, 18) mit wenigstens zwei Punkten des Halteelements (4, 15, 22) verbindbar ist.

4. Orthese nach Anspruch 3, **dadurch gekennzeichnet, dass** das Befestigungsband (7, 12, 18) an einem der Punkte durch eine Öse (9, 17) geführt und um 180° umgelenkt ist.

5. Orthese nach Anspruch 4, **dadurch gekennzeichnet, dass** der umgelenkte Abschnitt des Befestigungsbands (7, 12, 18) mit einer Verbreiterung unverlierbar in der Öse (9, 17) gehalten ist.

6. Orthese nach einem der Ansprüche 1 bis 5, **dadurch gekennzeichnet, dass** das Halteelement (4, 15, 22) in seiner aufgespreizten Ausgangsform flach liegt.

7. Orthese nach einem der Ansprüche 1 bis 6, **dadurch gekennzeichnet, dass** das Haltelemente (4, 15, 22) eine textile Bandage ist.

8. Orthese nach Anspruch 7, **dadurch gekennzeichnet, dass** die textile Bandage (4, 15, 22) in ihrer aufgespreizten Ausgangsform zur Anpassung an das zur Beaufschlagung mit der Bandage (4, 15, 22) vorgesehene Körperteil vorgeformt ist.

9. Orthese nach einem der Ansprüche 1 bis 6, **dadurch gekennzeichnet, dass** das Haltelemente (4, 15, 22) aus dem Aufspreizteil (13, 21, 24) besteht, auf dem wenigstens ein zum Körperteil gerichtetes Polsterstück (27) angebracht ist.

## Claims

1. Orthosis with at least one holding element (4, 15, 22), which is designed without a stable shaped part adapted to a circumferential shape of the body part, for placing on a body part and securing the holding element (4, 15, 22) with at least one securing means (7, 12, 18), by which two ends of the holding element (4, 15, 22) bearing on the body part can be connected to each other with tensioning, the holding element (4, 15, 22) having at least one elastic expansion part (13, 21, 24), which pretensions the holding element (4, 15, 22) in such a starting shape that the ends of the holding element (4, 15, 22) jut out from the body part, and, with the securing means (7, 12, 18), the ends of the holding element (4, 15, 22) can be applied to the body part counter to the pretensioning of the expansion part (13, 21, 24), **characterized in that** the expansion part has a pretensioning force which is simply sufficient to tension the holding element in the starting shape, such that the expansion part does not perform the function of a shaped part adapted to the body part and does not perform a supporting function for the body part.

2. Orthosis according to Claim 1, **characterized in that** the securing means (7, 12, 18) is a securing band.

3. Orthosis according to Claim 2, **characterized in that** the securing band (7, 12, 18) can be connected to at least two points of the holding element (4, 15, 22).

4. Orthosis according to Claim 3, **characterized in that** the securing band (7, 12, 18) is guided at one of the points through an eyelet (9, 17) and turned back through 180°.

5. Orthosis according to Claim 4, **characterized in that** the turned-back portion of the securing band (7, 12, 18) is held with a widened area captively in the eyelet (9, 17).

6. Orthosis according to one of Claims 1 to 5, **characterized in that** the holding element (4, 15, 22) lies flat in its expanded starting shape.

7. Orthosis according to one of Claims 1 to 6, **characterized in that** the holding element (4, 15, 22) is a textile bandage.

8. Orthosis according to Claim 7, **characterized in that** the textile bandage (4, 15, 22), in its expanded starting shape, is pre-shaped to adapt to the body part on which the bandage (4, 15, 22) is intended to be placed.

9. Orthosis according to one of Claims 1 to 6, **characterized in that** the holding element (4, 15, 22) is composed of the expansion part (13, 21, 24), on which is placed at least one padded section (27) directed toward the body part.

## Revendications

1. Orthèse comprenant au moins un élément de maintien (4, 15, 22) qui est réalisé sans partie conformée stable adaptée à une forme périphérique de la partie corporelle, destinée à être appliquée sur une partie corporelle et à fixer l'élément de maintien (4, 15, 22) avec au moins un organe de fixation (7, 12, 18), au moyen duquel deux extrémités de l'élément de maintien (4, 15, 22) sont susceptibles d'être reliées l'une à l'autre sous tension en étant appliquées sur la partie corporelle, dans laquelle l'élément de maintien (4, 15, 22) comprend au moins une partie d'écartement élastique (13, 21, 24), qui précontraint l'élément de maintien (4, 15, 22) sous une forme de départ telle que les extrémités de l'élément de maintien (4, 15, 22) dépassent de la partie corporelle et que les extrémités de l'élément de maintien (4, 15, 22) peuvent être appliquées contre la partie corporelle avec l'organe de fixation (7, 12, 18) à l'encontre de la précontrainte de la partie d'écartement (13, 21, 24),
**caractérisée en ce que** la partie d'écartement présente une force de précontrainte qui suffit simplement à tendre l'élément de maintien jusque dans la forme de départ, de sorte que la partie d'écartement ne remplit pas la fonction d'une pièce conformée adaptée à la partie corporelle et ne remplit aucune fonction de soutien pour la partie corporelle.

2. Orthèse selon la revendication 1, **caractérisée en ce que** l'organe de fixation (7, 12, 18) est une bande de fixation.

3. Orthèse selon la revendication 2, **caractérisée en ce que** la bande de fixation (7, 12, 18) peut être reliée à au moins deux points de l'élément de maintien (4, 15, 22).

4. Orthèse selon la revendication 3, **caractérisée en ce que** la bande de fixation (7, 12, 18) est guidée à travers un oeillet (9, 17) à l'un des points et est renvoyée sur 180°.

5. Orthèse selon la revendication 4, **caractérisée en ce que** la portion renvoyée de la bande de fixation (7, 12, 18) est maintenue de façon imperdable dans l'oeillet (9, 17) avec un élargissement.

6. Orthèse selon l'une des revendications 1 à 5, **caractérisée en ce que** l'élément de maintien (4, 15, 22) repose à plat dans sa forme de départ écartée.

7. Orthèse selon l'une des revendications 1 à 6, **caractérisée en ce que** l'élément de maintien (4, 15, 22) est un bandage textile.

8. Orthèse selon la revendication 7, **caractérisée en ce que** le bandage textile (4, 15, 22) est préformé, dans sa forme de départ écartée, en vue de l'adaptation à la partie corporelle prévue pour l'application avec le bandage (4, 15, 22).

9. Orthèse selon l'une des revendications 1 à 6, **caractérisée en ce que** l'élément de maintien (4, 15, 22) est constitué par la partie d'écartement (13, 21, 24), sur laquelle est appliquée au moins une pièce de rembourrage (28) orientée vers la partie corporelle.
